# EUROPEAN PATENT SPECIFICATION

(11) **EP 1 074 630 B1**
(45) Date of publication and mention of the grant of the patent: **18.01.2012**
(21) Application number: 00116094.4
(22) Date of filing: 27.07.2000
(51) Int. Cl.: C12P 7/26, C12P 41/00, C12N 11/04, C12R 1/85

(54) **Microbial production of levodione**
Mikrobielle Herstellung von Levodione
Production microbienne de levodione

(30) Priority: 02.08.1999 EP 99115237
(43) Date of publication of application: 07.02.2001
(73) Proprietor: DSM IP Assets B.V., 6411 TE Heerlen (NL)
(72) Inventor: Fukuoka, Masatsuka, Iwata-gun, Shizuoka-ken, 437-1121 (JP); Hiraga, Koki, Kakegawa-shi, Shizuoka-ken, 436-0062 (JP); Sekihara, Toru, Fukuroi-shi, Shizuoka-ken, 437-0028 (JP)
(74) Representative: Schwander, Kuno

(56) References cited:
- EP-A- 0 864 600
- US-A- 4 072 715

## Description

The present invention relates to the microbial production of (6R)-2,2,6-trimethylcyclohexane-1,4-dione (hereinafter referred to as levodione), and particularly to a process for producing levodione of high purity and in high yield by the catalytic reaction of 2,6,6-trimethyl-2-cyclohexene-1,4-dione (hereinafter referred to as ketoisophorone) with a specific yeast.

Levodione has previously been prepared through the reduction of the carbon-carbon double bond in ketoisophorone by contacting said ketoisophorone with baker's yeast, such as *Saccharomyces cervisiae*, which functions as an enantioselective biocatalyst (Biotechnology of Vitamins, Pigments and Growth Factors, Ed. Erick J. Vandamme, page 71, Elsevier Applied Science, London and New York). However, baker's yeast is not suitable for use in the industrial production as the yields of levodione are too low.

Further problems associated with the production process using baker's yeast and which had to be overcome for developing an industrially feasible production process were:
1) The yeast cells could not be reused because of the short lifetime of the reaction activity of the yeast.
2) A complicated purification process was necessary because it was difficult to separate the yeast from the culture solution after the catalytic reaction.

As a result of further development US 4 072 715 discloses the conversion of ketoisophorone in levodione by a large number of microorganisms.

As a result of extensive further studies to increase the yield of levodione produced from ketoisophorone and to overcome the other problems it has been found that yeast strain *Saccharomyces rouxii (Zygosaccharomyces rouxii)* HUT 7191 (IFO 0494) can produce levodione much more efficiently from ketoisophorone than hitherto. Accordingly, the present invention provides a process for producing levodione characterized by contacting ketoisophorone with *Saccharomyces rouxii (Zygosaccharomyces rouxii)* HUT 7191 (IFO 0494) capable of converting ketoisophorone into levodione in water, a water-miscible organic solvent or a mixture of water and said water-miscible organic solvent containing at least one assimilable carbon source, followed by isolating the resulting levodione from the reaction medium after completion of the reaction.

It has further been found that by entrapping *Saccharomyces rouxii (Zygosaccharomyces rouxii)* HUT 7191 (IFO 0494) by certain techniques and using the so-immobilized yeast for said catalytic reaction, levodione of high purity can be produced in even higher yield. This represents a preferred feature of the present invention.

Ketoisophorone used as the starting material or "substrate" in the process of the present invention is a well-known substance, and can itself be readily synthesized by methods known from the literature, e.g. as reported in US Patent 3,960,966.

*Saccharomyces rouxii* HUT 7191, is listed as such in the HUT Catalogue which issued in 1987. However, the HUT Catalogue which issued in September 1999 reclassifies the microorganism as *Zygosaccharomyces rouxii* HUT 7191.

The yeast used in the process of the present invention may be pre-prepared prior to its use in said process by cultivating the yeast in a growth medium containing an assimilable carbon source, an assimilable nitrogen source, inorganic salts and the like, followed by separating the yeast cells from the medium by a conventional process, such centrifugation. The cultivation of the yeast is generally carried out in a temperature range from 20 to 40°C, preferably from 25 to 30°C, in a pH range from 3.0 to 6.0, preferably from 4.3 to 4.7, and for 6 to 48 hours, preferably for 20 to 30 hours, under aerobic conditions. Suitable assimilable carbon sources include sucrose, sorbitol, glucose and the like, of which glucose is preferred. Suitable assimilable nitrogen sources include ammonium sulfate, sodium nitrate, peptone, amino acids, corn steep liquor, malt extract, bran extract, yeast extract and the like. Suitable inorganic salts include magnesium sulfate, sodium chloride, calcium carbonate, dipotassium phosphate and the like.

The process of the present invention is carried out in water, a water-miscible organic solvent or a mixture of water and said water-miscible organic solvent. As the water-miscible organic solvent there is suitably used a lower (C₁₋₆-) alkanol, especially methanol, ethanol or propanol. However, water is preferably used as the solvent based on economy and ease of handling.

The process is generally carried out in a temperature range from 20 to 40°C, preferably from 25 to 30°C. An optimal narrower temperature range for any combinations of other reaction conditions can be determined by the skilled artisan. The pH is normally from 3.0 to 6.0, preferably from 4.0 to 5.0. The reaction can normally be terminated 2 to 40 hours after the initiation of the reaction, preferably after confirming that the ketoisophorone concentration in the reaction medium has reached a very low or even zero value using an analytical method such as gas chromatography. The initial ketoisophorone concentration is normally 0.3 to 2.0% by weight, preferably 0.9 to 1.0% by weight.

As an assimilable carbon source, which must be present in the reaction medium as an energy source for the yeast to produce levodione from the ketoisophorone, there may be used sucrose, sorbitol, glucose or the like, of which glucose is preferred. The initial concentration of such a carbon source in the medium is normally 2.0 to 8.0% by weight, preferably 2.5 to 3.5% by weight.

The process of the present invention can be conducted in a batchwise, semi-continuous or continuous manner. In one particular embodiment yeast is suspended in the solvent, i.e. in water, a water-miscible organic solvent or a mixture of both, and the suspension is introduced into a reactor. Ketoisophorone and glucose are then dissolved in further solvent, and the solution adjusted to a pH of 3.0 to 6.0, preferably 4.0 to 5.0, and added continuously to the suspension of yeast in the reactor. After starting aeration in the reaction medium, the addition of the solution of ketoisophorone and glucose to the yeast suspension in the reactor is continued, and the resulting solution is removed continuously from the reactor by filtration. The rates of addition and removal are adjusted to about the same value to maintain the same volume in the reactor. The catalytic reaction is generally carried out in a temperature range from 20 to 40°C, preferably from 25 to 30°C. As the medium for adjusting the pH there is suitably used aqueous sulphuric acid or sodium hydroxide. The time during which the ketoisophorone reacts with the yeast can vary according to the prevailing conditions. However, said time may be pre-determined by adjusting both the feeding rate and the removal rate so that ketoisophorone in the removed solution is continuously being depleted.

In one aspect of the catalytic reaction of ketoisophorone with yeast according to the process of the present invention, yeast which is in a floating state in water or an aqueous solvent, or yeast in an immobilized state may be used for a fluidized bed reactor or for a fixed bed reactor. Even if the activity of the yeast has decreased, however, reactivation of the yeast in the immobilized form can be performed easily, and levodione can be produced in good yield using the yeast in spite of its previous repeated use for the catalytic reaction. Therefore, employing yeast in the immobilized form can improve the productivity of levodione.

The process of the present invention may be conducted using a fluidized bed reactor or a fixed bed reactor. In addition, when immobilized yeast is to be used for the first catalytic reaction with ketoisophorone, it is preferable in general that activation of said immobilized yeast be performed before the reaction. Such activation can be carried out by contacting the immobilized yeast with a suitable nutrient medium with bubbling and stirring under suitable conditions for growth of the yeast.

### 1: Use of a fluidized bed reactor

Ketoisophorone and glucose are dissolved in the water or aqueous solvent. The resulting solution and the immobilized yeast are then introduced into the reactor. The catalytic reaction is initiated by aeration and agitation. The concentration of ketoisophorone is normally 0.3 to 2.0% by weight, preferably 0.9 to 1.0% by weight, and that of glucose normally 2.0 to 4.0% by weight, preferably 2.5 to 3.0% by weight. The catalytic reaction may be carried out in a temperature range from 20 to 40°C, preferably 25 to 30°C, and in a pH range from 3.0 to 6.0, preferably 4.3 to 4.7. The aeration rate (volume of air/volume of medium/minute: vvm) is suitably 0.5 to 3 vvm, preferably 1 to 2 vvm. The reaction time may vary depending on the conditions. However, reaction is normally stopped about 8 to 10 hours after the initiation of the reaction, preferably after confirming that the amount of ketoisophorone has been depleted.

For isolating the levodione upon completion of the catalytic reaction, only the resulting solution is removed from the reactor, followed by washing the immobilized yeast with water or an aqueous solvent. Then the levodione can be isolated from the combined solution and washings by conventional processes. Immobilized yeast can be reactivated by being washed with washing solvent, and can be used repeatedly thereafter for further catalytic reaction.

### 2: Use of a fixed bed reactor

Immobilized yeast is placed into the reactor. Ketoisophorone and glucose are dissolved in the water or aqueous solvent and the pH of the solution is adjusted to a value from 3.0 to 6.0, preferably 5.0. The concentration of ketoisophorone is normally 0.3 to 2.0% by weight, preferably 0.9 to 1.0% by weight, and that of glucose normally 2.0 to 4.0% by weight, preferably 2.5 to 3.0% by weight. The resulting solution is introduced into the reactor for the reaction with the immobilized yeast. The catalytic reaction is suitably carried out in a temperature range from 20 to 40°C, preferably 25 to 30°C. The reaction time may vary depending on the conditions. However, the reaction is normally stopped 24 to 48 hours after the initiation of the reaction, preferably after confirming that the amount of ketoisophorone has been depleted. The process after the termination of the catalytic reaction is the same as in the case, described above, where a fluidized bed reactor is used.

Methods for immobilizing yeast are known: see for example W. M. Fogarty et al., Microbial Enzymes and Biotechnology, 2nd Edition, Elsevier Applied Science, pp. 373-394 (1983), or Japanese Patent Publication No. 61, 265/1994.

Examples of carriers used for immobilizing yeast by the entrapment method include the following:
(i) High molecular weight gel of polysaccharide, such as sodium alginate, potassium carrageenan, gelatin and agar, which is extracted from natural products ;
(ii) Chitosan beads, which consist of high molecular weight polysaccharide derived from natural products;
(iii) Ceramic beads; and
(iv) Hydrophobic photo-crosslinkable resins which contain at least two ethylenic unsaturated linkages per molecule, polymerized by irradiation

Among the above-mentioned carriers, hydrophobic photo-crosslinkable resin polymerized by irradiation, i.e. of type iv above, is most preferably used.

Examples of hydrophobic photo-crosslinkable resins include the following:
(iv,a) Urethane adduct formed from the reaction between polypropylene glycol and (meth)acrylic acid;
(iv,b) Urethane adduct formed from the reaction between polyethylene glycol and (meth)acrylic acid;
(iv,c) Polyvinylalcohol and
(iv,d) A mixture of the urethane adducts (iv,a) and (iv,b).

Such hydrophobic photo-crosslinkable resins are available commercially, an example being. "BEL ENTG-3800" from Kansai Paint Co., Ltd., Hyogo-ken, Japan

An example of process for immobilizing yeast using the above-mentioned irradiation-polymerized hydrophobic photo-crosslinkable resin as the carrier comprises the following steps:
preparing a liquid-state mixture of (a) the hydrophobic photo-crosslinkable resins, (b) a photo-polymerization initiator, such as benzoin, acetone, benzoinmethylether, naphthol or 2-hydroxy-2-methylpropiophenone [C₆H₅COC(CH₃)₂OH], (c) a solvent to dilute the hydrophobic photo-crosslinkable resin, such as petroleum benzine, benzene, hexane, ethylene glycol or formaldehyde, and (d) yeast,
dripping said mixture containing the components (a), (b), (c) and (d) into an aqueous solvent containing a detergent such as sodium lauryl sulfate or a glycerol fatty acid ester, to form solid beads, at which stage the diameter of the beads, which can however vary, is normally about 0.1 to 5 mm, preferably about. 0.5 to 3.0 mm, and
irradiating the beads with rays of wavelength about 250 to 600 nm for 2 to 5 minutes.

The ratio of the components, i.e. (a) : (b) : (c) : (d), by weight in the starting liquid-state mixture is normally 100 : 0.1 - 5 : 10 - 200 : 0.001 - 50, respectively, but it is not restricted thereto.

An example of a process for purifying the levodione obtained by the catalytic reaction involved in the process of the invention comprises the following steps:
removing the solid impurities and yeast cells by filtering or centrifuging the mixture of the resulting solution and the washings,
contacting the resulting filtrate with an adsorption resin, such as a hydrophobic resin, and
eluting the adsorbed levodione from the resin with a hydrophilic organic solvent, e.g. a lower alcohol such as methanol or ethanol, or acetone.
Using such an approach, levodione of high purity can be obtained.

Examples of said hydrophobic resins are modified crosslinked polystyrene (SP-207) and crosslinked polystyrene (SP-800 and SP-850) (Mitsubishi Chemical Co., Tokyo, Japan). Among these resins, SP-850 is the most preferred because its adsorption capacity for levodione per unit volume is extremely high. Levodione in a solvent can be efficiently adsorbed onto a hydrophobic resin by contacting the hydrophobic adsorption resin with the solution having a volume which is 10 to 20 times the volume of the resin. When conducting the elution step, it is preferred that methanol of twice the volume of the resin be used as the elution solvent. Levodione eluted from the hydrophobic adsorption resin is crystallized by concentrating the pooled eluate followed by cooling the resulting concentrated eluate. Then the levodione crystals can be isolated by filtration or centrifugation and obtained in this way in high yield.

By means of the process of the present invention, levodione having a purity of more than 99% can be produced at a recovery rate of 70 to 80%.

The following Examples illustrate the present invention in more detail:

### Example 1

### Screening of Yeast

Commercially available baker's yeast (available from the Oriental Yeast Co., Ltd., Tokyo, Japan; used for comparative purposes) and the following yeasts obtained from public depositaries (ATCC, HUT or IFO) were each separately cultivated in a medium containing glucose, polypeptone and yeast extract at 30°C for 45 hours, and then the yeast cells in the culture were collected by centrifugation.
*Saccharomyces cerevisiae* ATCC 7754 (also used for comparative purposes),
*Saccharomyces rouxii* (*Zygosaccharomyces rouxii*) HUT 7191 (IFO 0494),
*Saccharomyces delbrueckii* HUT 7116 (*Saccharomyces unisporus* IFO 0298),
*Saccharomyces delbrueckii* (*Torulaspora delbrueckii*) HUT 7102,
*Saccharomyces willianus* HUT 7106,
*Zygosaccharomyces bailii* ATCC 11486,
*Candida tropicalis* IFO 1403

In each case collected yeast cells were suspended in ion exchanged water at a concentration of 80 g/l, and the pH of the aqueous medium was adjusted to 4.2. In a separate operation ketoisophorone and glucose were dissolved in ion exchanged water, both at a concentration of 17 g/l, and the pH of the aqueous medium was adjusted to 4.2. The yeast suspension was mixed with the aqueous solution containing the ketoisophorone and glucose in a volume ratio of 1:1, and then the reaction was carried out at 30°C for 16 hours. Upon completion of the reaction, concentrations of the levodione in the seven cases were analyzed by gas chromatography, from which the levodione production rate per unit yeast was calculated for evaluation. The results are shown in Table 1.

**Table 1**

| Yeast used for screening | Levodione production rate (g/kg yeast cells/h) |
|---|---|
| Baker's yeast (Oriental Yeast Co., Ltd. Tokyo, Japan) | 8.8 |
| *Saccharomyces cerevisiae* ATCC 7754 | 9.8 |
| *Saccharomyces rouxii (Zygosaccharomyces rouxii)* HUT 7191 (IFO 0494) | 25.7 |
| *Saccharomyces delbrueckii* HUT 7116 (*Saccharomyces unisporus* IFO 0298) | 16.7 |
| *Saccharomyces delbrueckii* (*Torulaspora delbrueckii*) HUT 7102 | 16.0 |
| *Saccharomyces willianus* HUT 7106 | 14.8 |
| *Zygosaccharomyces bailii* ATCC 11486 | 12.6 |
| *Candida tropicalis* IFO 1403 | 10.5 |

From the results it is clear that each of the yeasts used in the present invention can produce levodione at a higher rate (≥10.5, as high as 25.7) than baker's yeast (8.8) and *Saccharomyces cerevisiae* ATCC 7754 (9.8)

### Example 2 -

### Catalytic Reaction Using Immobilized Yeast

*Saccharomyces rouxii* (*Zygosaccharomyces rouxii*) HUT 7191 (IFO 0494) and baker's yeast were cultivated for 24 hours at 30°C in a medium containing in the given weight percents yeast extract (0.5%), polypeptone (1.0%), glucose (2.0%), KH₂PO₄ (0.5%) and MgSO₄ (0.2%) using a stirred fermentor. Since the glucose was depleted by the yeast cells in a growth phase about 12 hours after initiation of cultivation, glucose was added at a constant rate thereafter. The cells were collected from the culture by centrifugation and then suspended in ion exchanged water at a concentration of 100 g/l. A mixture of photo-crosslinkable resin (ENTG-3800) containing parabenzoquinone at 200 ppm had been prepared in advance and stored at room temperature for more than one week before use. The mixture of resin and parabenzoquinone, calcium alginate solution (1.8% by weight), the cell suspension and 2-hydroxy-2-methylpropiophenone were mixed in a weight ratio of 55.5: 22.2: 22.2: 0.1 respectively. The mixture was dripped into calcium chloride solution (3.0% by weight) through a syringe needle to produce solid beads having a diameter of 3 mm. The beads were separated from the calcium chloride solution, and were immediately irradiated with ultraviolet rays (360 nm) for 3 minutes. After irradiation, the beads were washed with ion exchanged water. The preparation of the immobilized yeast was thus completed.

A vertically elongated bubble tower reactor (5 liter) with baffle plates was used in each case for the reaction. The reactor was packed with 800 g of the respective immobilized yeast. The solution of 3.5 immobilized yeast volumes which contained yeast extract (0.1%), glucose (2.0%), KH₂PO₄ (0.2%) and MgSO₄7H₂O (0.1%) was added and aeration was begun at a rate of 7.0 l/min. to activate the yeast. The temperature of the immobilized yeast and the activation solution was maintained at 30°C and the pH was maintained at 4.5 by addition of aqueous sodium hydroxide solution during activation. The activation was completed after 24 hours, and only the activation solution was removed from the reactor. Then, 35 g of ketoisophorone and 75 g of glucose were added to the reactor, and the volume was brought to 3.5 l with ion exchanged water. Aeration was begun at a rate of 7.0 l/min., and the catalytic reaction was initiated. The temperature of the immobilized yeast and the reaction mixture was maintained at 30°C and the pH was maintained at 4.5 with 4.5N sodium hydroxide solution during the fermentation.

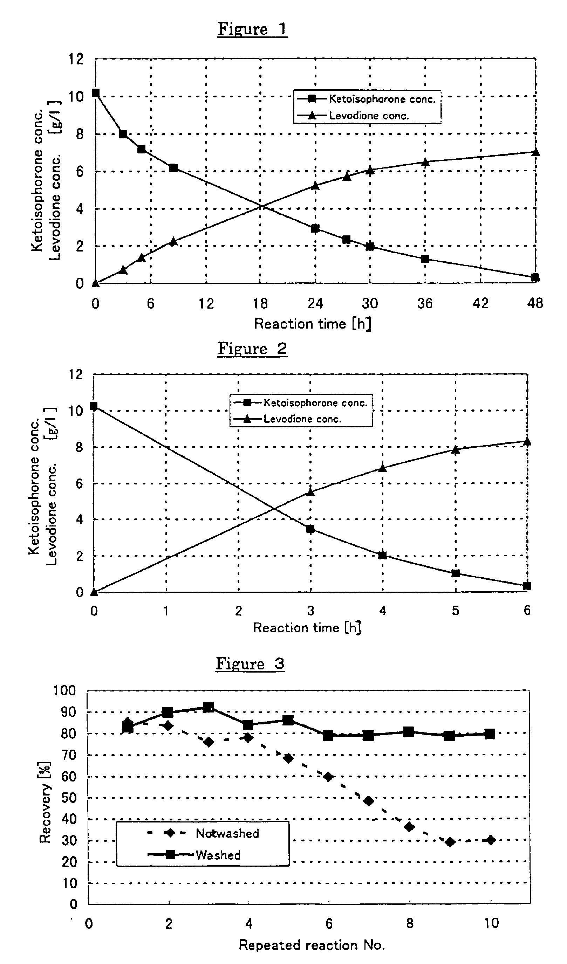

Figure 1 shows the time course of the changes in concentrations of ketoisophorone and levodione in the reaction solution using immobilized *Saccharomyces rouxii* (*Zygosaccharomyces rouxii*) HUT 7191.

Upon completion of the reaction, the reaction solution was removed and ketoisophorone and glucose were added thereto to perform the catalytic reaction once again. The reaction was repeated 10 times in the same manner, i.e. 10 reaction cycles were performed.

For comparison, Figure 2 shows the shifts in concentrations of ketoisophorone and levodione in the reaction solution using baker's yeast under the same conditions as described above.

Subsequently, after each completion of the repeated reaction, the reaction solution was removed from the reactor and water of 3.5 immobilized yeast volumes was added thereto and aeration was performed at a rate of 7.0 l/min for 10 minutes to wash the immobilized yeast. The washing solution was extracted thereafter, then ketoisophorone and glucose were added to the reactor to perform the catalytic reaction again. The catalytic reaction and the washing of the immobilized yeast were repeated 10 times in the same manner (10 cycles).

Figure 3 shows the shifts in recoveries of levodione to the added ketoisophorone from continuous fermentation using *Saccharomyces rouxii* (*Zygosaccharomyces rouxii*) HUT 7191, with or without incorporating a washing process.

### Example 3

### Industrial Scale Production of Levodione

*Saccharomyces rouxii* (*Zygosaccharomyces rouxii*) HUT 7191 (IFO 0494) was cultivated and a large amount of immobilized yeast was prepared by the same method as described in Example 2.

A vertically elongated bubble tower reactor with baffle plates (1 m³) was used for the catalytic reaction. The reactor was packed with 230 kg of immobilized yeast prepared in advance. The solution of 3.5 immobilized yeast volumes which contained in the given weight percents yeast extract (0.1%), glucose (2.0%), KH₂PO₄ (0.2%) and MgSO₄7H₂O (0.1%) was added thereto and aeration was begun at a rate of 0.4 Nm³/min to activate the yeast. The temperature of the immobilized yeast and the solution was maintained at 30°C and the pH at 4.5 with sodium hydroxide solution during the activation. The activation was completed after 24 hours, and the activating solution was removed from the reactor. Then, 9 kg of ketoisophorone and 30 kg of glucose were added to the reactor and the volume was brought to 1000 l with ion exchanged water. Aeration was begun at a rate of 0.4 Nm³/min, and the catalytic reaction was initiated. The temperature of the immobilized yeast and the solution was maintained at 30°C and the pH at 4.5 with sodium hydroxide solution during the reaction.

The reaction was completed once the amount of ketoisophorone had been depleted, and the resulting solution was removed from the reactor. Then, water of 3.5 immobilized yeast volumes was added to the reactor and aeration was performed at a rate of 0.4 Nm³/min for 15 minutes to wash the immobilized yeast. After the washing solution had been discharged, the same amounts described above of ketoisophorone and glucose were added to the reactor to carry out the reaction again. The reaction time is usually about 8 hours, however, if fermentation is carried out continuously more than 20 times, the production activity of the yeast will decline and the reaction time will be more than 10 hours resulting in a decrease in production efficiency. Therefore, activation of immobilized yeast was performed by the same method as mentioned above every 20 times. As a result, the activity of the yeast did not decline despite the immobilized yeast having been repeatelly used for the catalytic reaction 100 times.

The purification of levodione was carried out by crystallizing levodione with mixtures of the resultant solution and the extracted washing solution. The obtained mixtures were filtered by ultrafiltration, the filtration area being 5 m². The inflow rate was set at 250 l/h. The filtrate was loaded onto a 200 l column filled with 150 l of hydrophobic adsorption resin SEPABEADS SP-850 (Mitsubishi Chemical Co., Tokyo, Japan) at a rate of 200 l/h to adsorb levodione, ketoisophorone and byproducts such as actinol. For one adsorption operation, 3000 1 of filtrate were applied. The resin adsorbing levodione, ketoisophorone and byproducts in the column was heated to 50°C, and methanol solution, preheated at 50°C, was added to the column at a rate of 200 l/h to elute levodione, ketoisophorone and byproducts. For one elution operation, 200 l of methanol were applied. The methanol solution containing levodione, ketoisophorone and byproducts was concentrated 6-fold using a concentrator at 60°C under reduced pressure. The concentrate containing levodione, ketoisophorone and byproducts was cooled to 5°C and maintained at this temperature for more than 12 hours to crystallize levodione. The ketoisophorone and byproducts remained dissolved in methanol. The crystallized levodione was collected by filtration, washed with methanol to remove superficial ketoisophorone and byproducts, then dried. The production process was thereby completed, and levodione crystals having purity of more than 99% were obtained.

Using the above-described method, the reaction for producing levodione was repeated 100 times. Approximately 500 kg of levodione with an overall yield, based on the employed ketoisophorone, of about 70% and having a purity of more than 99% were produced.

### Example 4

### Evaluation of Levodione Productivity of Yeast in the Immobilized Form

A comparison of the levodione productivity of baker's yeast with that of *Saccharomyces rouxii* (*Zygosaccaromyces rouxii*) HUT 7191 (IFO 0494), which has a very high ability to produce levodione under free cell conditions, was performed, whereby both yeasts were in the immobilized state.

The method of measuring the activities of immobilized yeast was as follows:

Immobilized yeast was prepared by the same method as described in Example 1, and activation was conducted. Ketoisophorone and glucose were dissolved in ion exchanged water to a final concentration of 10 g/l and 50 g/l, respectively, and the pH was adjusted to 4.5. The immobilized yeast (30 g) was suspended in the ketoisophorone and glucose mixture (30 ml) prepared beforehand and the reaction was initiated at 30°C. The reaction was completed when the ketoisophorone had been depleted. Levodione concentrations in the reaction solution were analyzed by gas chromatography, and the productivity (g levodione/kg, yeast/h) of levodione by each yeast was then evaluated from the results, which are as follows:

| | Productivity |
|---|---|
| Baker's yeast (Oriental Yeast Co., Ltd., Tokyo, Japan) | 0.69 |
| *Saccharomyces rouxii* (*Zygosaccharomyces rouxii*) HUT 7191 (IFO 0494) | 4.88 |

## Claims

1. A process for producing (6R)-2,2,6-trimethylcyclohexane-1,4-dione **characterized by** contacting 2,6,6-trimethyl-2-cyclohexene-1,4-dione with yeast capable of converting 2,6,6-trimethyl-2-cyclohexene-1,4-dione into (6R)-2,2,6-trimethyl-cyclohexane-1,4-dione wherein the yeast is *Saccharomyces rouxii* HUT 7191 (IFO 0494), in water, a water-miscible organic solvent or a mixture of water and said water-miscible organic solvent containing at least one assimilable carbon source, followed by isolating the resulting (6R)-2,2,6-trimethylcyclohexane-1,4-dione from the reaction medium after completion of the reaction.

2. A process according to claim 1, **characterized in that** the yeast to be used is pre-prepared prior to its use in the process by cultivation in a growth medium containing an assimilable carbon source, an assimilable nitrogen source, inorganic salts and the like, followed by separation of the yeast cells from the medium.

3. A process according to claim 1 or 2, **characterized in that** the yeast used in the process for producing (6R)-2,2,6-trimethylcyclohexane-1,4-dione is immobilized yeast as obtained by an entrapment method.

4. A process according to claim 3, **characterized in that** the immobilized yeast has been obtained by entrapment using hydrophobic photo-crosslinkable resin having at least two ethylenic unsaturated linkages per molecule, polymerized by irradiation, as the carrier.

5. A process according to any one of claims 1 to 4, **characterized in that** 2,6,6-trimethyl-2-cyclohexene-1,4-dione is contacted with the yeast in a temperature range from 20 to 40°C, preferably from 25 to 35°C, and at a pH of from 3.0 to 6.0, preferably from 4.0 to 5.0.

6. A process according to any one of claims 1 to 5, **characterized in that** it is conducted using a fluidized bed reactor or a fixed bed reactor.

## Patentansprüche

1. Verfahren zur Herstellung von (6R)-2,2,6-Trimethylcyclohexan-1,4-dion, **dadurch gekennzeichnet, dass** man 2,6,6-Trimethyl-2-cyclohexen-1,4-dion mit Hefe, die fähig ist, 2,6,6-Trimethyl-2-cyclohexen-1,4-dion in (6R)-2,2,6-Trimethylcyclohexan-1,4-dion umzuwandeln, in Wasser, einem mit Wasser mischbaren organischen Lösungsmittel oder einer Mischung aus Wasser und dem mit Wasser mischbaren organischen Lösungsmittel, enthaltend mindestens eine assimilierbare Kohlenstoffquelle, in Kontakt bringt, wobei es sich bei der Hefe um Saccharomyces rouxii HUT 7191 (IFO 0494) handelt, und anschließend das erhaltene (6R)-2,6,6-Trimethylcyclohexan-1,4-dion nach Abschluss der Reaktion aus dem Reaktionsmedium isoliert.

2. Verfahren nach Anspruch 1, **dadurch gekennzeichnet, dass** die zu verwendende Hefe vor ihrer Verwendung in dem Verfahren **dadurch** vorbereitet wird, dass man sie in einem Wachstumsmedium, das eine assimilierbare Kohlenstoffquelle, eine assimilierbare Stickstoffquelle, anorganische Salze und dergleichen enthält, kultiviert und anschließend die Hefezellen aus dem Medium abtrennt.

3. Verfahren nach Anspruch 1 oder 2, **dadurch gekennzeichnet, dass** es sich bei der Hefe, die in dem Verfahren zur Herstellung von (6R)-2,2,6-Trimethylcyclohexan-1,4-dion verwendet wird, um eine immobilisierte Hefe, wie sie durch ein Einschlussverfahren erhalten wird, handelt.

4. Verfahren nach Anspruch 3, **dadurch gekennzeichnet, dass** die immobilisierte Hefe durch Einschluss unter Verwendung eines hydrophoben lichtvernetzbaren Harzes mit mindestens zwei ethylenisch ungesättigten Bindungen pro Molekül, das durch Bestrahlung polymerisiert wird, als Träger erhalten wird.

5. Verfahren nach einem der Ansprüche 1 bis 4, **dadurch gekennzeichnet, dass** das 2,6,6-Trimethyl-2-cyclohexen-1,4-dion mit der Hefe in einem Temperaturbereich von 20 bis 40°C, vorzugsweise von 25 bis 35°C, und bei einem pH-Wert von 3,0 bis 6,0, vorzugsweise von 4,0 bis 5,0, in Kontakt gebracht wird.

6. Verfahren nach einem der Ansprüche 1 bis 5, **dadurch gekennzeichnet, dass** es unter Verwendung eines Wirbelbettreaktors oder eines Festbettreaktors durchgeführt wird.

## Revendications

1. Procédé de production de (6R)-2,2,6-triméthylcyclohexane-1,4-dione, **caractérisé par** la mise en contact de la 2,6,6-triméthyl-2-cyclohexène-1,4-dione avec une levure capable de transformer la 2,6,6-triméthyl-2-cyclohexène-1,4-dione en (6R)-2,2,6-triméthylcyclohexane-1,4-dione, la levure étant Saccharomyces rouxii HUT 7191 (IFO 0494), dans de l'eau, un solvant organique miscible à l'eau ou un mélange d'eau et dudit dissolvant organique miscible à l'eau contenant au moins une source de carbone assimilable, suivie de l'isolement de la (6R)-2,2,6-triméthylcyclohexane-1,4-dione résultante du milieu réactionnel à la fin de la réaction.

2. Procédé selon la revendication 1, **caractérisé en ce que** la levure à utiliser est préparée avant son utilisation dans le procédé par culture dans un milieu de croissance contenant une source de carbone assimilable, une source d'azote assimilable, des sels minéraux et similaires, suivie de la séparation des cellules de levure du milieu.

3. Procédé selon la revendication 1 ou 2, **caractérisé en ce que** la levure utilisée dans le procédé de production de (6R)-2,2,6-triméthylcyclohexane-1,4-dione est une levure immobilisée telle qu'obtenue par une méthode de piégeage.

4. Procédé selon la revendication 3, **caractérisé en ce que** la levure immobilisée a été obtenue par piégeage en utilisant une résine photoréticulable hydrophobe ayant au moins deux liaisons éthylèniquement insaturées par molécules, polymérisée par irradiation, en tant que support.

5. Procédé selon l'une quelconque des revendications 1 à 4, **caractérisé en ce que** la 2,6,6-triméthyl-2-cyclohexène-1,4-dione est mise en contact avec la levure dans une gamme de températures de 20 à 40°C, de préférence de 25 à 35°C, et à un pH de 3,0 à 6,0, de préférence de 4,0 à 5,0.

6. Procédé selon l'une quelconque des revendications 1 à 5, **caractérisé en ce qu'**il est effectué en utilisant un réacteur à lit fluidisé ou un réacteur à lit fixe.
